# EUROPEAN PATENT APPLICATION

(11) **EP 2 107 071 A2**
(43) Date of publication of application: **07.10.2009**
(21) Application number: 09165284.2
(22) Date of filing: 09.02.2005
(51) Int. Cl.: C07K 16/30, A61K 39/395, A61P 35/00

(54) **Anti-EpCAM immunoglobulins**

(30) Priority: 13.02.2004 US 778915
(62) Divisional of application: 05707293.6
(71) Applicant: Micromet AG, 81477 München (DE)
(72) Inventor: Peters, Malte, 81477 Munich (DE); Locher, Mathias, 81477 Munich (DE); Prang, Nadja, 81477 Munich (DE); Quadt, Cornelia, 81477 Munich (DE)
(74) Representative: Dehmel, Albrecht

(57) **Abstract**

The invention relates *inter alia* to a method of treating tumorous disease in a human patient by administering to the patient a human immunoglobulin specifically binding to the human EpCAM antigen, the immunoglobulin exhibiting a serum half-life of at least 15 days, the method comprising the step of administering the immunoglobulin no more frequently than once every week, preferably no more frequently than once every two weeks.

SEQUENCE LISTING

<110>  Micromet AG

<120>  Anti-EpCAM Immunoglobulins

<130>  MIC-022 PCT/EP DIV 1

<140>  unknown
<141>  2009-07-13

<150>  US 10/778,915
<151>  2004-02-13

<160>  2

<170>  PatentIn version 3.3

<210>  1
<211>  457
<212>  PRT
<213>  artificial sequence

<220>
<223>  Anti-EpCAM Heavy Chain

<400>  1

Glu Val Gln Leu Leu Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Met Gly Trp Gly Ser Gly Trp Arg Pro Tyr Tyr Tyr Tyr
            100                 105                 110

Gly Met Asp Val Trp Gly Gln Gly Thr Thr Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Asp Glu Leu
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>  2
<211>  214
<212>  PRT
<213>  artificial sequence

<220>
<223>  Anti-EpCAM Light Chain

<400>  2

Glu Leu Gln Met Thr Gln Ser Pro Ser Ser Leu Ser Ala Ser Val Gly
1               5                   10                  15

Asp Arg Val Thr Ile Thr Cys Arg Thr Ser Gln Ser Ile Ser Ser Tyr
            20                  25                  30

Leu Asn Trp Tyr Gln Gln Lys Pro Gly Gln Pro Pro Lys Leu Leu Ile
        35                  40                  45

Tyr Trp Ala Ser Thr Arg Glu Ser Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Ser Ala Thr Tyr Tyr Cys Gln Gln Ser Tyr Asp Ile Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

## Description

### A. Field of the Invention

The present invention relates to methods of treating tumorous diseases using immunoglobulin molecules. In particular, the present invention relates to methods of treatment involving anti-EpCAM immunoglobulin molecules. The invention further relates to uses of such immunoglobulins in the production of medicaments. The invention further relates to immunoglobulin molecules which can be used treating tumorous diseases as well as compositions comprising such immunoglobulin molecules.

### B. Related Art

In designing a therapeutic regimen involving the administration of immunoglobulin molecules, there are several factors which must be considered. On the one hand, the therapeutic immunoglobulin must be administered to a patient in a quantity sufficient to elicit the desired therapeutic effect. This effect should be realized upon initial treatment and should continue to be realized to as great an extent as possible as the immunoglobulin is progressively cleared from the patient's body in the time between two consecutive administrations. On the other hand, the amount of immunoglobulin administered must not be so great as to cause adverse and/or toxic side effects in the patient.

A problem therefore arises when the maximum dose of an immunoglobulin which can be tolerated without causing side effects (**m**aximum **t**olerated **d**ose, or "MTD") limits the amount of immunoglobulin to a single-dose level which is insufficient to maintain, over time, the minimum level of immunoglobulin needed to ensure continued efficaciousness. In such a scenario, it becomes impossible to maintain the "serum trough level" needed to ensure a continued therapeutic effect until the next administration of immunoglobulin. The "serum trough level" of a medicament refers generally to the lowest concentration that medicament is allowed to reach at any time in a patient's blood without loss of therapeutic effect. It represents, then, the minimum amount of medicament which must always be present in the patient's blood in order for any therapeutic benefit to be realized.

Several approaches exist for maintaining a desired serum trough level of a therapeutic immunoglobulin. One approach is to increase the initial dose of the immunoglobulin to the patient. However, this approach has the disadvantage that the level of therapeutic immunoglobulin which is safe for the patient is likely to be exceeded and the patient is thus likely to experience adverse and/or toxic side effects.

Another approach is to increase the frequency of administration of the therapeutic immunoglobulin. However, an increased frequency of administration stands to severely detract from the patient's quality of life, as multiple and frequent visits to the clinic become necessary. This is especially the case when the illness to be treated is still in the early stages, and the patient would otherwise be able to lead a normal life.

Further, an increased application frequency implies a larger total amount of therapeutic immunoglobulin which is needed for a total therapeutic regimen. As such, an increased application frequency implies higher total costs associated with a given regimen of therapy as compared to a regimen of therapy in which the therapeutic immunoglobulin is administered less frequently.

In the event that the therapeutic immunoglobulin to be administered is specific for an antigen which is present in both healthy and diseased tissue, the antigen being more prevalent in diseased than in healthy tissue, it becomes all the more crucial to develop a treatment regimen which takes the above points into consideration. Here, the danger is especially great that too high or too frequent dosages will lead to undesired interaction between the therapeutic immunoglobulin and the antigen to which the therapeutic immunoglobulin specifically binds. These immunoglobulin-healthy tissue interactions stand to lead to adverse and/or toxic side effects which can complicate a regimen of therapy using the immunoglobulin.

One such antigen present both in healthy and diseased human tissue is the **ep**ithelial **c**ell **a**dhesion **m**olecule ("EpCAM", also called 17-1A antigen, KSA, EGP40, GA733-2, ks 1-4 and esa). EpCAM is a surface glycoprotein expressed by cells of simple epithelia and tumorous cells derived therefrom. Although the EpCAM molecule is displayed on the surface of cells from healthy tissue, its expression is up-regulated in malignant tissue. EpCAM serves to adhere to epithelial cells in an oriented and highly ordered fashion (Litvinov, J Cell Biol. 1997, 139, 1337-1348). Data from experiments with transgenic mice and rats expressing human EpCAM on their epithelia suggest that EpCAM on normal tissue may however not be accessible to systemically administered antibody (McLaughlin, Cancer Immunol. Immunother., 1999, 48, 303-311). Upon malignant transformation of epithelial cells the rapidly growing tumor cells are abandoning the high cellular order of epithelia. Consequently, the surface distribution of EpCAM becomes less restricted and the molecule better exposed on tumor cells. Due to their epithelial cell origin, tumor cells from most carcinomas still express EpCAM on their surface.

In the past, EpCAM has been shown to be a rewarding target for monoclonal immunoglobulin treatment of cancer, especially in patients with minimal residual disease suffering from disseminated tumor cells that may cause later solid metastases and thus worsen the patients' prognosis. In patients with minimal residual colorectal cancer, a murine monoclonal immunoglobulin specific for the EpCAM molecule decreased the 5-year mortality rate by 30% as compared to untreated patients, when applied systemically in five doses within four months after surgery of the primary tumor (Riethmüller, Lancet 343 (1994), 1177-83). More recently, strong EpCAM over-expression has been reported in about 40% patients with breast cancer and is associated with poor overall and disease-free survival (Spizzo et al., Int. J. Cancer 98 (2002), 883-8). Most recently, EpCAM expression was analyzed in 3,722 patients. It was found that EpCAM expression is very common in epithelial tumors, such expression having been observed in more than 88% of tumor samples. Specifically, EpCAM expression was observed in 94.1% of ovarian cancers, 94% of colon cancers, 92.3% of stomach cancers, 90.1% of prostrate cancers and 70.9% of lung cancers.

One example of a (murine) monoclonal antibody recognizing EpCAM is Edrecolomab (Panorex) (Koprowski, Somatic Cell Genet. 1979, 5, 957-971 and Herlyn, Cancer Res., 1980, 40, 717-721; incorporated by reference in its entirety). However, the first administration of Panorex during adjuvant immunotherapy of colon cancer led to the development and exacerbation of Wegener's granulomatosis suggesting that Panorex should be applied cautiously in a patient with autoimmune disease (Franz, Onkologie 2000, 23, 472-474; incorporated by reference in its entirety). The limitations of Panorex are the rapid formation of human anti-mouse antibodies (HAMA), the limited ability to interact by its murine IgG2a Fcγ receptor with human immune effector mechanisms and the short half-life in circulation (Frodin, Cancer Res., 1990, 50, 4866-4871; incorporated by reference in its entirety). Furthermore, the murine antibody caused immediate-type allergic reactions and anaphylaxis upon repeated injection in patients (Riethmüller, Lancet 1994, 343, 1177-1183, Riethmüller, J Clin Oncol., 1998, 16, 1788-1794 and Mellstedt, Annals New York Academy of Sciences 2000, 910, 254-261; each incorporated by reference in their entirety).

ING-1 is another known anti-EpCAM immunoglobulin (Lewis, Curr. Op. Mol. Ther. 5, 433-6, 2003; incorporated by reference in its entirety). ING-1 is a mouse-human chimeric IgG1 immunoglobulin currently in Phase I/II clinical studies of patients with advanced epithelial tumors. While a dose of 1 mg/kg of immunoglobulin was found to provide the greatest effect in mice which had been pre-injected with human tumor cells, this dosage led to pancreatitis in 2 out of 2 human patients with adenocarcinomas (amylase and lipase elevation with abdominal pain), precluding further dose escalation. The MTD for ING-1 was found to be only 0.3 mg/kg body weight, applied intravenously every 3 weeks. Considering that the ING-1 half-life at this dosage was about 31 hours and assuming that the average adult weighs 75 kg and has about 4.25 liters of blood, the serum level of ING-1 after 21 days *(i.e.,* after 16.25 half-lives) would have decreased to below 7 x 10⁻⁵ µg/mL blood, more than four orders of magnitude less than the 1 µg/mL serum level found to be necessary for maximum cytolytic effects. The MTD of ING-1 therefore prevents the necessary plasma trough level of anti-EpCAM immunoglobulin from being maintained.

There therefore exists a need for a treatment regimen involving anti-EpCAM antibodies which can be used for the treatment of cancer. Correspondingly, an aim of the present invention is to provide a treatment regimen involving anti-EpCAM immunoglobulins which overcomes the problems as outlined above.

### SUMMARY OF THE INVENTION

The foregoing need is met by a method of treating tumorous disease in a human patient by administering to said patient a human immunoglobulin specifically binding to the human EpCAM antigen, said immunoglobulin exhibiting a serum half-life of at least 15 days, said method comprising the step of administering said immunoglobulin no more frequently than once every week.

Several advantageous effects are realizable by using an anti-EpCAM immunoglobulin with a serum half-life of at least 15 days. Most importantly, this relatively long serum half-life implies that the anti-EpCAM immunoglobulin administered as part of the inventive method will not be cleared from the blood as rapidly as another immunoglobulin with a shorter half-life, say that of IMG-1 as discussed above. Assuming, then, that an anti-EpCAM immunoglobulin fulfilling the requirements of the immunoglobulin to be used in the method of the invention and an anti-EpCAM immunoglobulin not fulfilling these requirements are both administered to a human simultaneously and in identical absolute amounts, more of the former immunoglobulin will persist in the serum after a given time than the latter immunoglobulin. In a converse sense, the enhanced persistence in the serum allows less of the anti-EpCAM immunoglobulin used in the inventive method to be administered at one time than would be possible for another anti-EpCAM of shorter serum half life while still maintaining a certain predetermined serum trough level, *i*.*e*., while ensuring that the total serum concentration of therapeutic agent never drops below the minimum level determined to be necessary for continued efficacy between two consecutive administrations. This has the advantageous effect that less of the anti-EpCAM immunoglobulin of the method of the invention need be applied in any given dose, thereby eliminating the possibility of or at least mitigating any adverse and/or toxic side effects.

The relatively long half-life of the anti-EpCAM immunoglobulin as used in the method of the invention also implies that administration need not take place too frequently, thereby increasing the quality of life for the patient and reducing total cost of therapy.

That the anti-EpCAM immunoglobulin used in the method of the invention is a human immunoglobulin reduces or even eliminates the possibility of an undesired immune response mounted by the patient's immune system against the administered immunoglobulin. As such the problems associated with human anti-mouse antibodies ("HAMAs") observed when using many murine or even murine-human chimeric immunoglobulin molecules in therapy do not pose a problem according to the inventive method.

While not being bound by theory, the inventors believe that an anti-EpCAM immunoglobulin as used in this aspect of the invention elicits a therapeutic effect based on at least one of two different mechanisms in vivo. One mechanism is known as antibody-dependent cellular cytotoxicity ("ADCC"). In ADCC, a cell ("target cell") which is coated with immunoglobulin is killed by a cell ("effector cell") with Fc receptors which recognize the Fc portion of the immunoglobulin coating the target cell. In most cases, the effector cells participating in ADCC are natural killer ("NK") cells which bear on their surface either the Fc receptor Fc-□-RIII and/or the molecule CD16. In this way, only cells coated with immunoglobulin are killed, so the specificity of cell killing correlates directly with the binding specificity - here, EpCAM - of the immunoglobulin coating such cells.

Another mechanism by which the immunoglobulin as used in this aspect of the invention elicits a therapeutic effect is known as complement-dependent cytotoxicity ("CDC"). In CDC, two identical immunoglobulins bind to two identical antigens (for example, here EpCAM) on the surface of a target cell such that their respective Fc portions come into close proximity to one another. This scenario attracts complement proteins, among them complement proteins clq and c3 and c9, the latter of which creates a pore in the target cell. The target cell is killed by this perforation. At the same time, the target cell/s also become/s decorated at other locations on its/their surface/s in a process called opsonization. This decoration attracts effector cells, which then kill the target cell/s in a manner analogous to that described above in the context of the ADCC mechanism.

By virtue of the long half life of the immunoglobulin used in the method according to this aspect of the invention, the benefit of one or both of the above mechanisms may be exploited for a longer time, and at higher levels, than possible using an anti-EpCAM immunoglobulin with a shorter half life.

According to this aspect of the invention, the anti-EpCAM immunoglobulin is administered to a patient no more frequently than once every week, preferably no more frequently than once every two weeks. In this respect, the advantageously long serum half-life of the anti-EpCAM immunoglobulin is exploited. In the event that the administration takes place once every week, only small amounts of immunoglobulin will need to be administered in any one administration, as more than half of the previously administered immunoglobulin will still persist in the blood of the patient. This is because one week is less than the approximately 15-day half life of the immunoglobulin previously administered.

In the event that the administration takes place about once every two weeks, the dosing frequency according to the inventive method corresponds approximately to the half life of the immunoglobulin. As such, the serum level of this immunoglobulin in the interim between two consecutive administrations will never have decreased by more than about one-half its amount immediately following the respective previous administration. This means that the dosage of any given administration need be no higher than the amount required to lead, immediately after administration, to approximately two times the predetermined serum trough level reached by the time of the next administration.

Generally, one may define two phases of administration: a first "loading phase" in which one or more loading doses is/are administered so as to reach a certain steady plasma level of immunoglobulin, and a subsequent "maintenance phase" in which multiple maintenance doses are administered so as to maintain the desired immunoglobulin plasma level. The loading dose(s) is/are typically administered in higher amount and/or in more frequent succession than the later maintenance doses, thus keeping the duration of the loading phase to a minimum.

According to present dosing regimens not corresponding to the present aspect of the invention, the medical practitioner is faced with two choices: Either the anti-EpCAM immunoglobulin is administered in a high enough initial amount to ensure, following its rapid clearance from the body, that the serum trough level is maintained before the next administration (in which case the high initial dose is likely to cause adverse and/or toxic side effects such as pancreatitis); or the anti-EpCAM immunoglobulin is administered in a low enough initial amount to avoid adverse and/or toxic side effects (in which case the serum level of anti-EpCAM immunoglobulin drops below the serum trough level before the next administration, leading to a loss of therapeutic effect). The compromise is to increase the frequency of administration of the low dosage, leading to a significant loss of quality of life for the patient.

In contrast, the method according to this aspect of the invention strikes a balance in which, on the one hand, individual doses may be administered in amounts which do not lead to adverse and/or toxic side effects and, on the other hand, the amount of therapeutic immunoglobulin in the serum does not drop below the serum trough level required for continued therapeutic effect between consecutive administrations. The rhythm of at least about one week between two consecutive administrations, preferably at least about two weeks between two consecutive administrations, allows this balance while not unduly impairing the quality of life for the patient.

According to one embodiment of the invention, the serum level of the anti-EpCAM antibody still present from a previous administration is checked in the patient's blood prior to effecting a next administration. In this way, the medical practitioner can avoid re-administering the anti-EpCAM immunoglobulin too early, as would for example be the case if there still existed ample anti-EpCAM immunoglobulin in the patient's blood from the previous administration. Accidental overdosing, which may lead to adverse and/or toxic side effects, is thus avoided for an anti-EpCAM antibody for which the exact half life is not yet known. At the same time, the medical practitioner gains valuable knowledge regarding the clearance rate of the anti-EpCAM immunoglobulin used from such an interim measurement, which in any case occurs at least two weeks following a respective prior administration. This knowledge can be valuable in fine-tuning the further administration schedule. Such fine tuning may advantageously entail waiting significantly longer than one week, or preferably longer than about two weeks, between consecutive administrations, thereby further increasing the patient's quality of life.

Advantageously, such interim checking of serum level of the anti-EpCAM immunoglobulin in the patients blood may be performed in the following manner. First, the medical practitioner may determine, after a period of at least one week following a respective last administration of said immunoglobulin but prior to a respective next administration of said immunoglobulin, the serum level of said immunoglobulin still present in the blood of said patient, thereby obtaining an intermediate serum level value for said immunoglobulin. This intermediate serum level value for said immunoglobulin is then compared with a predetermined serum trough level value for said immunoglobulin. If the intermediate serum level value for said immunoglobulin is found to be well above the predetermined serum trough level for said immunoglobulin, then the medical practitioner may advantageously elect to wait even longer for the serum level of the anti-EpCAM immunoglobulin to decrease further. At this time, the above steps may be repeated in order to obtain a new intermediate serum level of said immunoglobulin, which will then have decreased to a value closer to the predetermined serum trough level. In any case, one should not wait so long that the intermediate serum level determined for the immunoglobulin sinks below the predetermined serum trough level for that immunoglobulin. When the medical practitioner establishes, possibly by repeated cycles of waiting, determining intermediate serum level, and comparing this intermediate serum level to the predetermined serum trough level for a particular anti-EpCAM immunoglobulin, that the intermediate serum level of this immunoglobulin has decreased to within a certain percentage of said serum trough level, the respective next administration of the anti-EpCAM immunoglobulin may be effected to bring the serum level of the anti-EpCAM immunoglobulin back up to an appropriate level for the next round of clearance. Advantageously, this certain percentage may correspond to a serum level which is within 15%, preferably within 10%, most preferably within 5% of the predetermined serum trough level for the particular anti-EpCAM immunoglobulin used.

Advantageously, intermediate immunoglobulin serum level may be measured by any method known to one of ordinary skill in the art, for example, by immunoassay. For example, an immunofluorescence assay, a radioimmunoassay or an enzyme-linked immunosorbent assay - ELISA assay may be used for this purpose, the latter being preferred.

In a preferred embodiment of this aspect of the invention, the human anti-EpCAM immunoglobulin is administered no more frequently than once every two weeks. In an especially preferred embodiment, administration takes place in intervals of two weeks, wherein each subsequent dose is equivalent in amount to the first dose administered, *i.e.,* all doses are made in the same amount. Administration in this way is sufficient to maintain a serum level of immunoglobulin which never drops below the predetermined serum trough level required for a beneficial therapeutic effect of this immunoglobulin, while at the same time avoiding, or largely avoiding adverse and/or toxic side effects.

However, in another embodiment it is also envisioned that administration frequencies of more than, or much more than two weeks are possible. In the event that the immunoglobulin is administered in time intervals greater than two weeks, the amount of antibody administered at any time subsequently to the initial dose should be greater than an initial dose made in expectation of a subsequent administration in two weeks. The amount by which such a subsequent dose administered after more than two weeks may be greater than a dose administered after two weeks may be determined on a case by case basis, for example by means of pharmacokinetic simulations (*e.g.,* with WinNonlin 4.0.1 (Pharsight Corporation, USA; 2001) such as those described in the examples appended to the foregoing description. One of ordinary skill in the art understands how to construct and/or apply such simulations. Such simulations are advantageously constructed such that, after a respective administration, the level of anti-EpCAM immunoglobulin in the patient's serum is not allowed to drop below the serum trough level determined to be necessary for therapeutic efficacy.

The long serum half life of the human anti-EpCAM immunoglobulin ensures that over this longer period between administrations, say three or even four weeks or any intermediate period from 2 to 5 weeks, the predetermined serum trough level required for therapeutic efficacy is maintained. In other words, the long serum half life of the human anti-EpCAM immunoglobulin (i.e., about 15 days) ensures that a significant quantity of this immunoglobulin will still be present in the serum from a respective previous administration. As a result, less of the human anti-EpCAM immunoglobulin with the half life of about 15 days need be applied than would be necessary for an antibody without such a long serum half life. This reduces the risk of adverse and/or toxic side effects.

It should be noted that such protracted administration schemes - and the multiple advantages associated therewith (see above) - would be impossible with an anti-EpCAM immunoglobulin with a short half life, while still maintaining therapeutic effect (for example with the anti-EpCAM immunoglobulin ING-1, for which the serum half life in humans ranging from 17 to 31 hours has been measured). To ensure that at least the predetermined serum trough level amount of such an anti-EpCAM immunoglobulin would persist in the serum until the following administration, so much anti-EpCAM immunoglobulin would have to be administered that adverse and/or toxic side effects would very likely be encountered. On the other hand, avoiding such adverse and/or toxic side effects by administering less of such a short-lived anti-EpCAM immunoglobulin would lead to loss of therapeutic effect at some point between the two administrations when the amount of immunoglobulin persisting in the blood sinks below the serum trough level.

Of course, if determined clinically necessary or advantageous, a human anti-EpCAM immunoglobulin with a serum half life of about 15 days may in a further embodiment be administered in time intervals of less that two weeks, say in intervals of 1 week or any intermediate period from 1 week to 2 weeks. While such a scenario does not fully exploit the long serum half life of about 15 days, there are nevertheless clinical situations in which such an administration may be desirable. In order to avoid an unwanted accumulation of the human anti-EpCAM immunoglobulin in the patient over time, it is preferable here to reduce the amount of human anti-EpCAM administered in these shorter intervals relative to the amount which would need to be administered in a bi-weekly administration rhythm. The amount by which such a subsequent dose administered after less than two weeks must be less than a dose administered after two weeks may be determined on a case by case basis, for example by means of pharmacokinetic simulations such as those described in the examples appended to the foregoing description. One of ordinary skill in the art understands how to construct and/or apply such simulations. Such simulations are advantageously constructed such that, after a respective administration, the level of anti-EpCAM immunoglobulin in the patient's serum is not allowed to drop below the serum trough level determined to be necessary for therapeutic efficacy.

According to one embodiment, the administration may be intravenous, intraperitoneal, subcutaneous, intramuscular, topical or intradermal. Alternatively, a combination of these administration methods may be used as appropriate. Further envisaged are co-administration protocols with other compounds, e.g., bispecific antibody constructs, targeted toxins or other compounds, which act via T cells or other compounds such as antineoplastic agents which act via other mechanisms. The clinical regimen for co-administration of the anti-EpCAM immunoglobulin may encompass co-administration at the same time, before or after the administration of the other component.

Advantageously, the tumorous disease is chosen from breast cancer, epithelial cancer, hepatocellular carcinoma, cholangiocellular cancer, stomach cancer, colon cancer, prostate cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, pancreas cancer, gall bladder cancer, a cancer of the bile duct, esophagus cancer, a cancer of the salivatory glands or a cancer of the thyroid gland.

In another embodiment, the disease may also be a minimal residual disease, preferably early solid tumor, advanced solid tumor or metastatic solid tumor, which is characterized by the local and non-local reoccurrence of the tumor caused by the survival of single cells.

In an especially preferred embodiment of this aspect of the invention, the tumorous disease is prostate cancer or breast cancer. Here, it is especially preferred that the human anti-EpCAM immunoglobulin administered is one which comprises an immunoglobulin heavy chain with an amino acid sequence as set out in SEQ ID NO: 1 and an immunoglobulin light chain with an amino acid sequence as set out in SEQ ID NO: 2. When such a human anti-EpCAM immunoglobulin is administered, it is preferable that it be administered in a respective amount of dosage of 1-7 mg/kg body weight, even more preferably 2-6 mg/kg body weight about once every two weeks.

A further aspect of the invention provides a use of a human immunoglobulin specifically binding to the human EpCAM antigen, said immunoglobulin exhibiting a serum half life of at least 15 days, for the preparation of a medicament for treating tumorous diseases. Alternatively, a composition comprising such an immunoglobulin may be used for preparing the above medicament. The medicament may then be advantageously administered according to the dosage schedule outlined above for the method of treatment of a tumorous disease.

According to one embodiment of this aspect of the invention, the medicament prepared is suitable for administration by an intravenous, an intraperitoneal, a subcutaneous, an intramuscular, a topical or an intradermal route. Alternatively, administration may take place by a combination of more than one of these routes as appropriate. Further envisaged are co-administration protocols with other compounds, e.g., bispecific antibody constructs, targeted toxins or other compounds, which act via T cells or other compounds such as antineoplastic agents which act via other mechanisms. The clinical regimen for co-administration of the anti-EpCAM immunoglobulin may encompass co-administration at the same time, before or after the administration of the other component.

Advantageously, the tumorous disease is breast cancer, epithelial cancer, hepatocellular carcinoma, cholangiocellular cancer, stomach cancer, colon cancer, prostate cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, pancreas cancer, gall bladder cancer, a cancer of the bile duct, esophagus cancer, a cancer of the salivatory glands or a cancer of the thyroid gland.

In another embodiment, the disease may also be a minimal residual disease, preferably early solid tumor, advanced solid tumor or metastatic solid tumor, which is characterized by the local and non-local reoccurrance of the tumor caused by the survival of single cells.

In a further aspect, the invention relates to a human immunoglobulin specifically binding to the human EpCAM antigen, characterized in that said immunoglobulin exhibits a serum half-life of at least 15 days after administration to a human patient. The advantages associated with such a long serum half life have been elaborated above, within the framework of such an antibody's use in a method of treatment for tumorous diseases. It is preferred that the immunoglobulin exhibits a serum half life of 20 days, 19 days, 18 days, 17 days, 16 days or 15 days. Especially preferred is a serum half life of about 15 days.

According to a preferred embodiment of the invention, the half life of the human immunoglobulin is 15 days and the human immunoglobulin comprises an immunoglobulin heavy chain with an amino acid sequence as set out in SEQ ID NO: 1 and an immunoglobulin light chain with an amino acid sequence as set out in SEQ ID NO: 2.

A further aspect of the invention provides a composition comprising a human anti-EpCAM immunoglobulin as described above. Such a composition may advantageously be administered to a human patient as part of a regimen of therapy for treating a disease. In view of the prevalence of expression of the EpCAM molecule in tumorous diseases, it is especially preferred that such a composition may be administered as part of a therapeutic regimen aimed at treating such a tumorous disease. Tumorous diseases which may advantageously be treated by administration of such a composition according to this aspect of the invention include breast cancer, epithelial cancer, hepatocellular carcinoma, cholangiocellular cancer, stomach cancer, colon cancer, prostate cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, pancreas cancer, gall bladder cancer, a cancer of the bile duct, esophagus cancer, a cancer of the salivatory glands or a cancer of the thyroid gland.

In another embodiment, the disease may also be a minimal residual disease, preferably early solid tumor, advanced solid tumor or metastatic solid tumor, which is characterized by the local and non-local reoccurrence of the tumor caused by the survival of single cells.

It is within this aspect of the invention that such tumorous diseases may be treated either alone or in combination, a combination of such diseases having for example arisen due to metastatic spreading of a primary tumorous disease to lead to a or multiple secondary tumorous disease(s).

As used herein, the terms "antibody", "antibody molecule", "img" and "img molecule" are to be understood as equivalent. Where appropriate, any use of the plural implies the singular, and any use of the singular implies the plural.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
- **FIG. 1**: Dosing schemes for Phase I cohorts
- **FIG. 2**: Plasma concentration of anti-EpCAM immunoglobulin vs. time, per cohort
- **FIG. 3**: pharmacokinetic parameters of patient cohorts after single dose of anti-EpCAM immunoglobulin
- **FIG. 4**: pharmacokinetic parameters of patient cohorts after multiple doses of anti-EpCAM immunoglobulin
- **FIG. 5**: Schematic representation of three-compartment model
- **FIG. 6**: Peak and trough plasma levels of anti-EpCAM immunoglobulin with target trough level of 30 µg/mL
- **FIG. 7**: Peak and trough plasma levels of anti-EpCAM immunoglobulin with target trough level of 10 µg/mL
- **FIGS. 8A-F**: Immunohistological staining of EpCAM-expressing tissues
- **FIG. 9**: Median values of EpCAM semi-quantitative histological scores in patients with various liver diseases.

### DETAILED DESCRIPTION OF THE INVENTION

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1: Acquisition of pharmacokinetic data measured in the phase I study

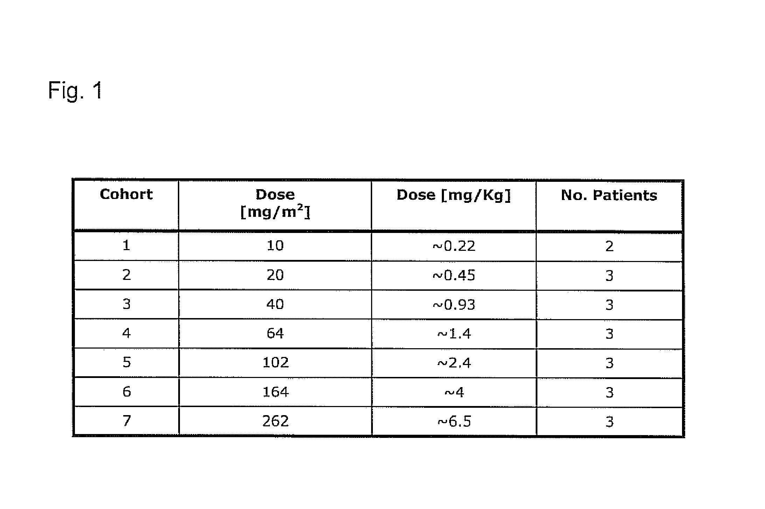

**Cohorts.** The pharmacokinetics of an anti-EpCAM immunoglobulin characterized by SEQ ID NOs: 1 and 2 (hereinafter "Anti-EpCAM") were investigated in patients with hormone refractory prostate cancer following two single intravenous infusions at a time interval of 14 days. The administered dosages were 10, 20, 40, 64, 102, 164 and 262 mg/m² body surface area. Two or three patients at each dose level were treated on day 1 and day 15. Blood samples were taken at 29 - 31 sampling time points from day 1 to day 70 (56 days after second administration). The serum concentrations of Anti-EpCAM were measured by a specific ELISA method. The ELISA was set up as a typical sandwich ELISA, in which a rat anti-Anti-EpCAM antibody was used as the capture antibody and a chicken anti-Anti-EpCAM antibody as the detection antibody (as described in Sambrook, Molecular Cloning, Cold Spring Harbor Laboratory Press). The dosing schemes used for the Phase I patient cohorts are shown in FIG. 1. The symbol "∼" in column 3 of FIG. 1 denotes that the values calculated for the doses, carrying the units mg/kg, are the result of average doses (taken over the number of patients in the respective cohort) divided by the average body weight (also taken over the number of patients in the respective cohort). As such, a respective dosage value represents the quotient of two average values.

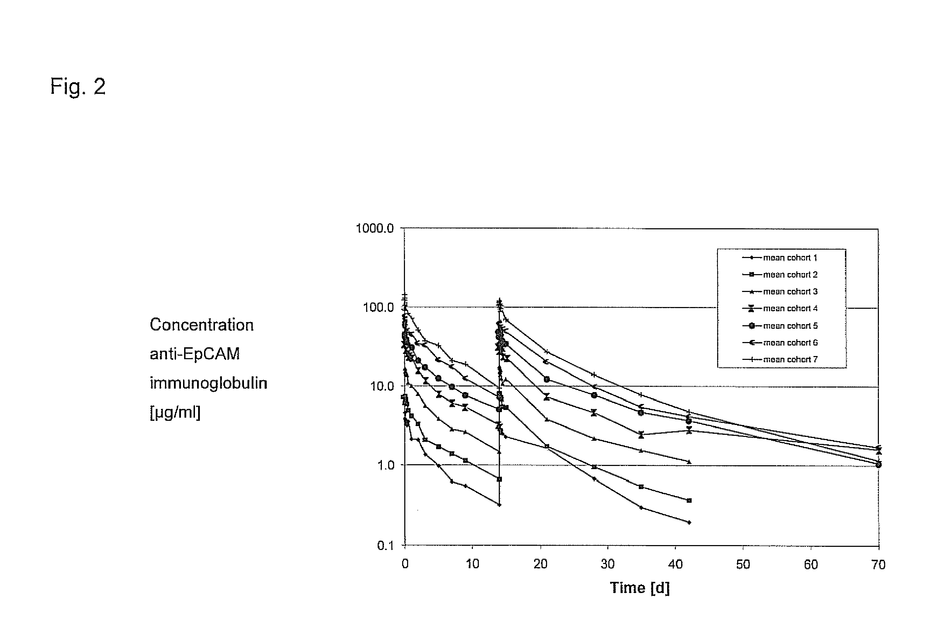

**Serum Concentrations.** The serum levels of Anti-EpCAM (mean values ± SD from 2-3 determinations) were measured in the individual patients after two single intravenous infusions of Anti-EpCAM. A comparison of the individual profiles within the single cohorts are presented in FIG. 2. The mean concentration/time profiles (arithmetic means) obtained for all dose groups of patients with hormone refractory prostate cancer following two single intravenous infusions at an time interval of 14 days are shown in FIG. 2.

**Simplified Dosage Scheme.** Patients received a personalized dosage, which was calculated in mg Anti-EpCAM /m² body surface area. Due to the consistency of the serum profiles observed for the different patients within one dose group, it was analyzed whether a simplification of the dosage scheme would be feasible. For this purpose, the profiles of the cohorts 5, 6 and 7 were normalized to an equal total dose of 500 mg and the results compared with respect to the variability of the serum levels.

For the 9 patients, the dose normalization to 500 mg total dose led to serum levels varying by a mean coefficient (% CV) of 26.6%. The coefficient of variation ranged from 14.8 to 67.3%, the highest variation was observed at lower serum levels. Based on these results, a simplification of the dose regimen to a total dose was considered to be feasible.

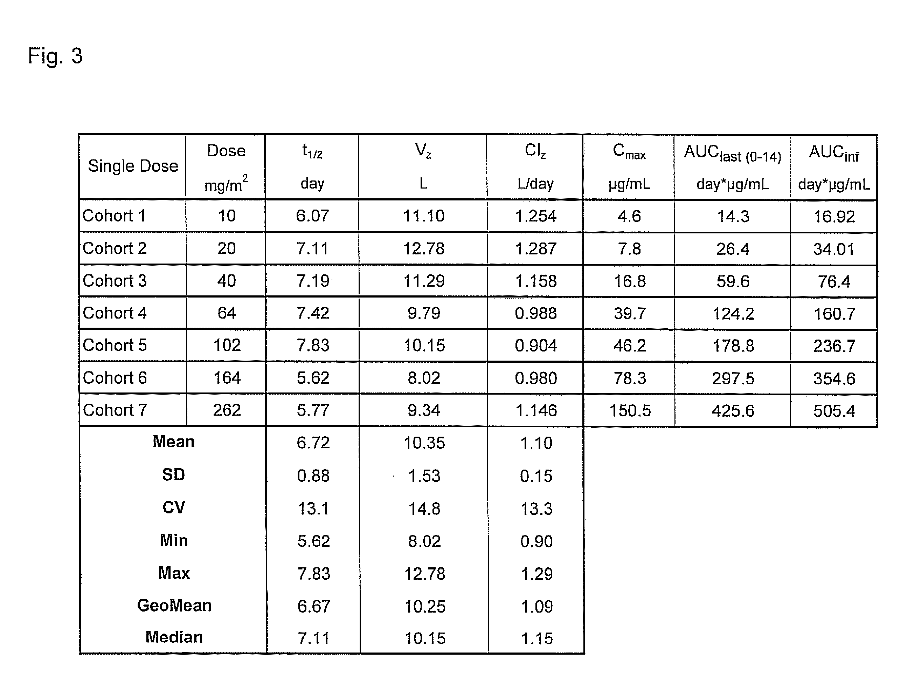

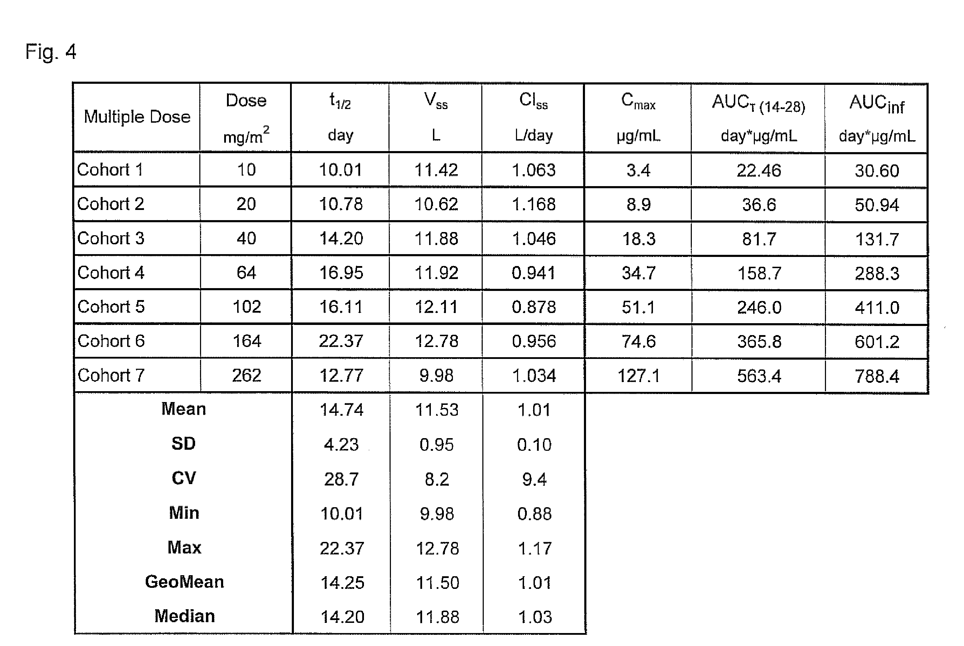

**Pharmacokinetics: Non-Compartmental Evaluation.** A summary of the main pharmacokinetic parameters (arithmetic means) calculated for patients of all seven cohorts with hormone refractory prostate cancer following the **first** intravenous infusion (single dose) of Anti-EpCAM is presented in FIG. 3. The main pharmacokinetic parameters (arithmetic means) of Anti-EpCAM after the **second** intravenous administration (multiple dose) on day 14 is shown in FIG. 4.

Definitions of terms used in FIGS. 3 and 4 are as follows. Cₘₐₓ refers to the maximum (measured) concentration. AUC_{τ}refers to the area under the concentration/time curve (AUC) observed in one dose interval (τ = 14 days) calculated with the trapezoidal rule from 14 to 28 days (for multiple dose). AUC_{INF} refers to the AUC calculated using the trapezoidal rule from 0 hours to infinity according to the formula AUC∞ = AUCz + Cz/ke. t½ refers to the mean apparent terminal half-life (ln2/λz), wherein the term "mean" refers to the averaging of multiple values determined for serum half life; the term "apparent" refers to extrapolation of a curve fit to selected pharmacokinetic values to an infinite time point such that the amount of immunoglobulin present in a patient's serum at infinite time decays asymptotically to zero; and the term "terminal" refers to this infinite time point. The parameter τ is a standard pharmacokinetic parameter used as a constant multiplication factor, and the parameter z denotes any time point z. Clₛₛ refers to the total body clearance, calculated according to the formula Dose/AUC. Vₛₛ refers to the apparent volume of distribution. Vz refers to the mean volume of distribution. CL refers to the mean volume of clearance.

The mean apparent terminal half-life (t½) was determined to be 6.72 ± 0.88 days after single dose (calculated from 7 - 14 days) and 14.74 ± 4.23 days after multiple dose administration (calculated from the last three sampling points, *i*.*e*., 28 - 42 days or 35 - 70 days). The differing half-life values are due to the clearly longer observation period after the second dose, measured half life values becoming more accurate the longer values are measured due to improved goodness of curve fit. As such, the value for t½ of 14.74 ± 4.23 days represents the more accurate value for t½, since it was measured over a long period of time.

After the first administration a Vz of 10.4 L and a mean volume of clearance of 1.1 L/day was determined. These data are well in accordance with the results calculated for the second dose with a mean Vz of 11.5 L and a mean volume of clearance (CL) of 1.0 L/day. Moreover these data are well comparable between all dose groups (coefficient of variation from 8.2 to 14.8%). As a result, dose-dependency was observed neither for the parameter Vz nor for the parameter CL.

**Dose Linearity.** The dose relationship regarding the parameters Cₘₐₓ, AUCₗₐₛₜ₍₀₋₁₄₎ / AUC_{τ(14-28)} and AUC_{inf} were determined. For all parameters (Cₘₐₓ, AUCₗₐₛₜ₍₀₋₁₄₎ / AUC_{τ(14-28)} and AUC_{inf}) one may assume a dose-linear increase in the investigated dose range.

### Pharmacokinetics: Compartmental Evaluation.

The compartmental analysis was based on two different models requiring a constant infusion of the drug. For the assessment of the best compartmental model the data obtained from cohort 6 relating to mean concentration vs. time was chosen. For both evaluations the profile after the second dose was applied due to the longer observation time after administration.

In order to investigate the best fit, the following compartmental models were employed:
- 2-Compartment Evaluation
- 3-Compartment Evaluation
   With both models, an evaluation was possible, however, a clearly better fit was obtained with the 3-compartment analysis. The congruence between observed Y and predicted Y was noticeably better after 3-compartmental analysis. For this reason, all further evaluations were performed on the basis of this 3-compartmental model.
   The pharmacokinetics of Anti-EpCAM were investigated in patients following intravenous short-term infusion of 10, 20, 40, 102, 164 and 262 mg/m² body surface area. Two or three patients per cohort were treated. Blood samples were taken over a time period of 42 or 70 days. The serum concentrations of Anti-EpCAM were measured by an ELISA method. Complete serum profiles up to 42 or 70 days could be obtained and evaluated for all patients.
   Volume of clearance and volume of distribution showed no dose dependency and no major differences after the first and the second dose. Based on the data from 7 cohorts, dose-linearity for the parameters Cₘₐₓ, AUC_{τ1}, AUCₗₐₛₜ and AUC_{inf} in the investigated dosage range can be assumed.
   Compartmental analysis showed a third-order decline of Anti-EpCAM serum concentrations with half-lives of 0.565 days (t_{½a}), 3.78 (t_{½β}) and 13.3 days(t_{½λz}).
   As expected from the terminal half-life (approximately two weeks), the simulations of various dose regimens produced the best results for a biweekly design. The simulation of a weekly dose led to an accumulation whereas the administration every 4 weeks resulted in a decrease of Anti-EpCAM serum levels. In view of reaching the target trough levels as fast as possible, a loading dose with the double amount compared to the maintenance dose is recommended.

### Example 2: Modeling of Anti-EpCAM dosing strategy based on measured data obtained in the phase I study

The dosage regimen and treatment duration selected for this study are based on pharmacokinetic modeling of the results of the phase I/II clinical study with Anti-EpCAM in patients with prostate cancer. The objective of the simulations was to find a dosing schedule for Anti-EpCAM to achieve serum trough levels of 10 and 30 µg/mL, respectively.

Based on preclinical experiments, serum trough levels of 10 µg/mL are expected to be effective for anti-tumor activity of Anti-EpCAM. However, it cannot be ruled out that higher doses might be more effective. Therefore, a second dose, calculated to achieve serum trough level of 30 µg/mL, is to be evaluated in clinical trials. No additional toxicity is expected with this serum trough concentration as Cmax and AUC values do not exceed the ones observed in phase I clinical studies.

Due to the better fit, all simulations were based on the 3-compartmental evaluation data from cohorts 5 to 7.

The aim of the simulations was to assess the optimum administration scheme and the required dose in consideration of frequency (weekly, biweekly, every 4 weeks), different trough levels (10 µg/mL, 30 µg/mL Anti-EpCAM) and to evaluate the benefit of a loading dose of Anti-EpCAM.

As expected from the terminal half-life value of ca. two weeks, the biweekly dosage regimen led to the best results. Applying an administration frequency of 7 days and 28 days, the simulation resulted in an accumulation or a slight decrease of serum levels, respectively. The application of a loading dose (LD) led to immediate attainment of the required trough levels. The following doses and corresponding minimum and maximum serum levels were simulated for intravenous administration of Anti-EpCAM.

**Administration every 14 days.** As expected from the terminal half-life of Anti-EpCAM, the biweekly administration resulted in simulated profiles with constant Cₘᵢₙ and Cₘₐₓ values and therefore can be regarded as the recommended dosage regimen. Therefore, the biweekly model was chosen for the calculation of the required dosages leading to target trough levels of 10 and 30 µg/mL of Anti-EpCAM.

The initial parameters for the calculations were gained by a compartmental evaluation.
- Study data: Pharmacokinetic measurements obtained in the Prostate Cancer Phase I/II Study.
- Software: WinNonlin 4.0.1 (Pharsight Corporation, USA; 2001)
- Model: PK Model 19 (3 compartment IV-Infusion, macro-constants, no lag time, 1st order elimination, uniform weighting).

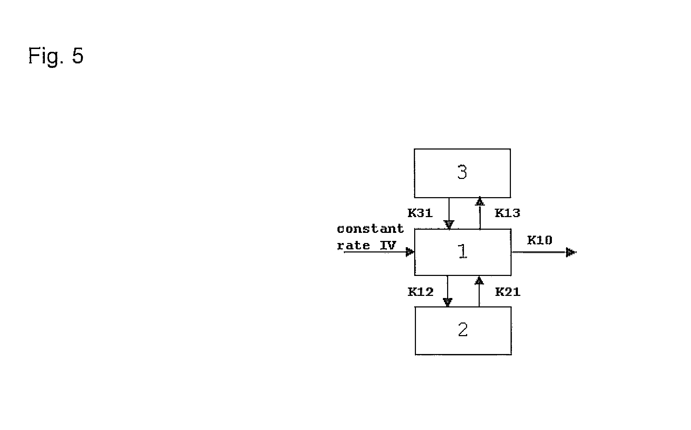

   FIG. 5 is a schematic representation of the three compartment model, where '1' represents the central compartment and '2' and '3' represent two different peripheral compartments. The central compartment is in immediate equilibrium with the plasma. The peripheral compartment requires some time to reach an equilibrium with the central compartment following an administration of a drug. K13, K31, K12, K21, K10 are the respective velocity constants, wherein the order of the numerals 13, 31, etc. denotes the direction of passage of Anti-EpCAM.

The simulations were extended to a period of 120 days, although the original study data were limited to a period of 70 days. The simulations were based on a loading phase (i.e., administration of drug on days 1, 8, and 15) and a maintenance phase (i.e., administration of drug on days 29 and every 14 days thereafter):
- Group A (low dose): loading phase of 2 mg Anti-EpCAM/kg body weight weekly (days 1, 8, 15), followed by 23 maintenance doses of 2 mg Anti-EpCAM/kg body weight every second week
- Group B (high dose): loading phase of 6 mg Anti-EpCAM/kg body weight weekly (days 1, 8, 15), followed by 23 maintenance doses of 6 mg Anti-EpCAM/kg body weight every second week.
   The doses intended in this study lead to pharmacokinetic parameters (*i*.*e*., Cₘₐₓ and AUC) that do not exceed those measured with the highest doses administered to patients in the phase I study. Loading phases and maintenance phases have been calculated using pharmacokinetic modeling to achieve targeted serum trough concentrations within a short period of time and to avoid maximum plasma concentrations that would exceed the ones assessed in the phase I study.

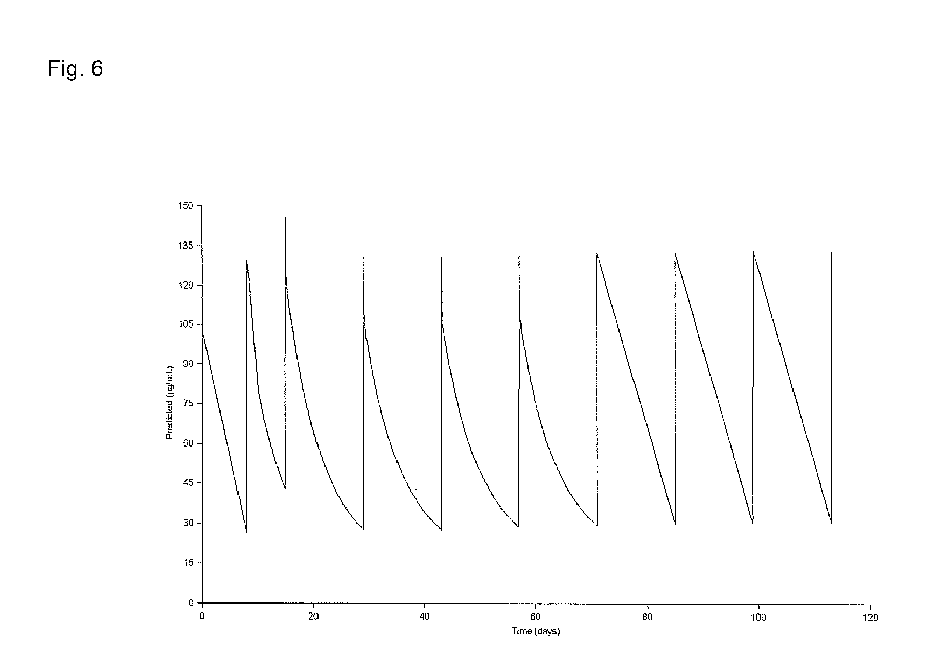

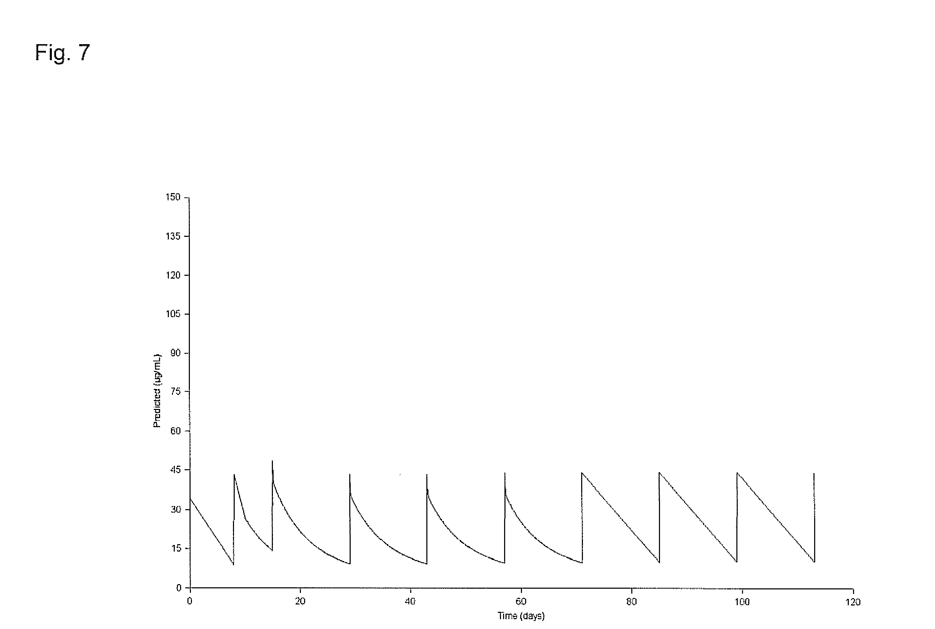

FIG. 6 shows a simulation of a biweekly administration described above of Anti-EpCAM including a loading phase with a target serum trough level of 30 µg/mL. FIG. 7 shows a simulation of a biweekly administration of Anti-EpCAM described above including a loading phase with a target serum trough level of 10 µg/mL.

FIGS. 6 and 7 show the respective administrations of drug over a time scale of 120 days. Peak and trough serum concentrations can be seen, the peak levels being represented by the upper portions of the curve and trough levels being represented by the lower part of the curves. Graphs represent the simulations to reach the above-mentioned different trough levels of 10 and 30 µg/ml, respectively. As can be seen from the figures, the peak and trough serum concentrations are different in the two simulations.

### Example 3: Anti-EpCAM toxicity data, comparison with ING-1, extrapolation

The following describes adverse events (AE) observed for the various patient cohorts. For the purposes of the following, an AE is defined as any untoward medical occurrence in a patient or clinical investigation subject to whom a pharmaceutical product is administered and which does not necessarily have a causal relationship with this treatment. It could therefore be any unfavorable and unintended sign (including abnormal laboratory findings), symptom, or disease temporally associated with the use of the investigational product, whether or not considered related to the investigational product.

Adverse drug reactions (*i*.*e*., AEs considered at least possibly related to study drug by the investigator) were graded by the investigator according to NCI Common Toxicity Criteria (CTC, version 2.0). For adverse drug reactions not listed on the NCI CTC tables, the general definitions for grading of severity of adverse events were to be followed. Accordingly, a "mild" AE describes a symptom which is barely noticeable to the patient. It does not interfere with the patient's usual activities or performance and/or it is of no clinical consequence. A "moderate" AE interferes with the usual activities of the subject and is sufficient enough to make the subject uncomfortable. It is of some clinical consequence; treatment for symptoms may be required. A "severe" AE is an event which causes severe discomfort and may be of such severity that the study treatment should be discontinued. The subject is unable to work normally or to carry out usual activities and/or the AE is of definite clinical consequence. Treatment for symptoms may be required. A "serious adverse event" (SAE) is defined as any untoward medical occurrence that, at any dose: Resulted in death, was life-threatening, required inpatient hospitalization or prolongation of existing hospitalization, resulted in persistent or significant disability/incapacity, or was a congenital anomaly/birth defect.

A total of 120 adverse events (AEs) regardless of relationship with study drug were reported in 19 (95%) patients during the treatment and the safety follow-up period of 28 days after the last infusion. More adverse events were reported in patients from cohort 6 (38 events) and in cohort 7 (35 events) compared to the lower dose cohorts (cohort 1: 7; cohort 2: 9; cohort 3: 12; cohort 4: 7; cohort 5: 12). The cohorts are set out in Fig.1, explained above in Example 1.

The most frequent treatment-emergent clinical AEs, regardless of the investigator's assessment of relation to study drug, were increase in body temperature (reported in 30% of all patients), nausea (30%), pyrexia (20%), diarrhea (15%), fatigue (15%), feeling cold (15%) and vomiting (15%). The most frequent treatment-emergent laboratory changes reported as adverse events, regardless of the investigator's assessment of relation to study drug, were elevated alkaline phosphatase (reported in 30% of all patients), lymphopenia (30%), elevated LDH (25%), PTT decrease (20%), hemoglobin decrease (20%), WBC disorders (15%), glycosuria (15%) and elevated transaminases (15%).

Most of the adverse events were mild (70%) or moderate (25%). Six severe adverse events (grade 3) were reported in four patients as follows: Elevated alkaline phosphatase in a patient with moderate (grade 2) value prior to treatment; Glycosuria in a patient with a known diabetes mellitus; One patient with decreased hemoglobin and RBC and weight loss; one patient with intervertebral disc herniation. None of the events was related to the study drug as assessed by the investigator. No grade 4 event was reported.

Four serious adverse events (SAE) were reported in 4 patients during the study period. One was considered possibly related to study medication by the investigator: a prolongation of hospitalization due to grade 1 fever after the 2nd infusion of Anti-EpCAM in a patient from Cohort 3 (40 mg/m² body surface area).

Clinical studies with the mouse-human chimeric, high-affinity (K_{D}: 2 x 10⁻⁹) anti-EpCAM antibody ING-1 resulted in pancreatitis at a dose of 1 mg/kg. These adverse events were dose dependent with a clear MTD. It is possible that the affinity of the immunoglobulin ING-1 towards the EpCAM antigen, higher by two orders of magnitude as compared to Anti-EpCAM, is related to the toxicity profile observed for ING-1. As the MTD of ING-1 (1 mg/kg) and the highest doses intended in the Anti-EpCAM protocol (6 mg/kg) are similar, it is expected that Anti-EpCAM, the immunoglobulin of the foregoing studies, has a significantly higher safety margin, possibly due to its much lower affinity.

### Example 4: EpCAM expression in disease

In order to assess the range of applicability of the method of treatment described herein, the expression of the human EpCAM antigen was studied in a number of different diseases. It is expected that the method of the invention may be efficaciously applied to any disease in which EpCAM expression is elevated in the disease state relative to the healthy state of a given tissue. In particular, special attention was paid to the synthesis of the EpCAM antigen in liver tissue.

**Patients and Tissues.** Overall 254 different liver tissue specimens were characterized by immunohistology for EpCAM and for relevant morphological parameters as outlined below. Different tumor samples, including 63 HCCs, 5 cholangiocarcinomas of the liver, and 30 dysplastic nodules (pre-malignant hepatocellular precursor lesions), as well as 5 normal liver specimens were analyzed. 33 biopsies were taken from patients with chronic hepatitis C, 27 from patients with chronic hepatitis B, and 28 from those with chronic alcoholic liver disease (ALD); 9 patients had autoimmune hepatitis (AIH). Liver tissues were obtained by biopsy using a Menghini needle and in the case of HCCs by resection or liver explantation. Tissues were immediately fixed in 4% neutral buffered formaldehyde and processed according to standard protocols.

**Morphological Evaluation.** Morphological evaluation was performed on the basis of sections stained with H&E (grading of carcinomas and chronic hepatitis). Grading of HCCs was performed as outlined in Nzeako et al., Cancer 76, 1995, 579-88. Non-neoplastic liver diseases were morphologically evaluated as follows: Necroinflammatory activity of chronic hepatitis B and C cases was analyzed by using the modified hepatic activity index as described in Ishak, Mod. Pathol. 7, 1994, 690-713.

**Immunohistological Evaluation.** Immunohistology was performed as previously described (Prange et al., J. Pathol. 201, 2003, 250-9) using the so-called ABC method and diaminobenzidine as the chromogen. Mouse monoclonal anti-human EpCAM antibody (clone VU-1D9; Novocastra, Newcastle, UK) was diluted 1/50 and applied after 30 min trypsin pre-treatment (0.1 %, pH 7.8). Immunohistology for cyclin D1 (DCS-6; 1:100; DAKO, Hamburg, Germany), p53 (FL-393; 1:50; Santa Cruz, Santa Cruz, USA), and ubiquitin (70458; 1:200; DAKO) was performed accordingly. Negative controls, including omission of the primary antibody were performed.

For evaluation of EpCAM staining in HCCs only intensity was graded semi-quantitatively (0 = negative, + (1) = weakly positive, ++ (2) = moderately positive, +++ (3) = strongly positive (at least equal intensity as bile duct staining)). Hepatocellular expression of EpCAM in non-neoplastic biopsy specimen was graded as follows: 0 = no hepatocellular staining; (+) (0.5) = few scattered positive hepatocytes, + (1) = small groups of hepatocytes along several or most septa or portal tracts, ++ (2) = large groups of positive hepatocytes around several or most portal tracts or septa and extending into midacinar zone, +++ (3) = extensive hepatocellular positivity, typically covering at least 50% of the acinus. Statistical evaluation was performed by using descriptive statistics (mean, median, maximum, frequency) and the correlation coefficient of Spearman. A level of p<0.05 was considered significant.

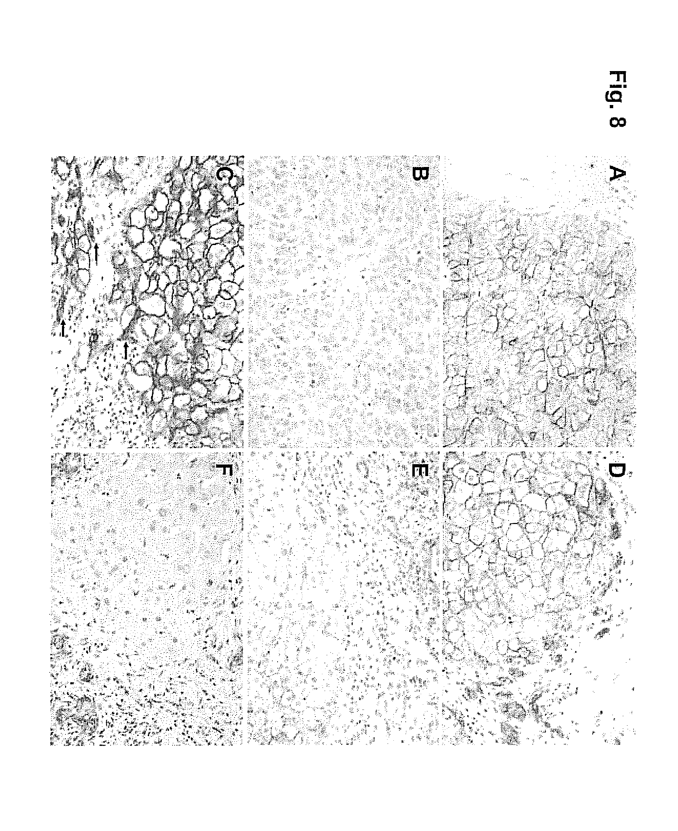

**Neo-expression of EpCAM in HCCs.** Normal liver tissue showed strong staining of all bile duct epithelia, while hepatocytes were completely negative (data not shown). Immunohistology for EpCAM showed specific membranous staining in 9 out of 63 analyzed HCCs (14.3 %; FIGS. 8A-F) and in all analyzed cholangiocarcinomas of the liver (n=5). In HCCs, expression ranged from weak to strong and appeared to be more frequent in moderately and poorly differentiated HCCs, while only one well differentiated HCC was positive. Also among 30 dysplastic nodules, which represent pre-malignant lesions, only 3 showed mild EpCAM expression. The same tissues were analyzed for numerous other tumor-relevant antigens and the expression data were submitted to correlative analyses. There was a moderate but significant positive correlation of EpCAM expression in HCCs with nuclear accumulation of p53 and ubiquitin (p<0.05), but not with the suspected upstream regulator cyclin D1.

### Hepatocellular neo-expression of EpCAM in chronic necroinflammatory liver disease.

Specific membranous positivity of hepatocytes was detected in a high percentage of the analyzed non-neoplastic liver tissues (FIGS. 8C-E). Marked positivity was found in cases with chronic hepatitis and to a lower extent in those with ALD. Furthermore, positivity of all ductular proliferations and also of single small cells dispersed in the periportal parenchyma (potential precursor cells) was noted. Hepatocellular positivity showed strong periportal/periseptal predominance and reached the intensity of bile duct staining in some of the cases. No specific reactivity for EpCAM was present in non-parenchymal liver cells in any of the cases.

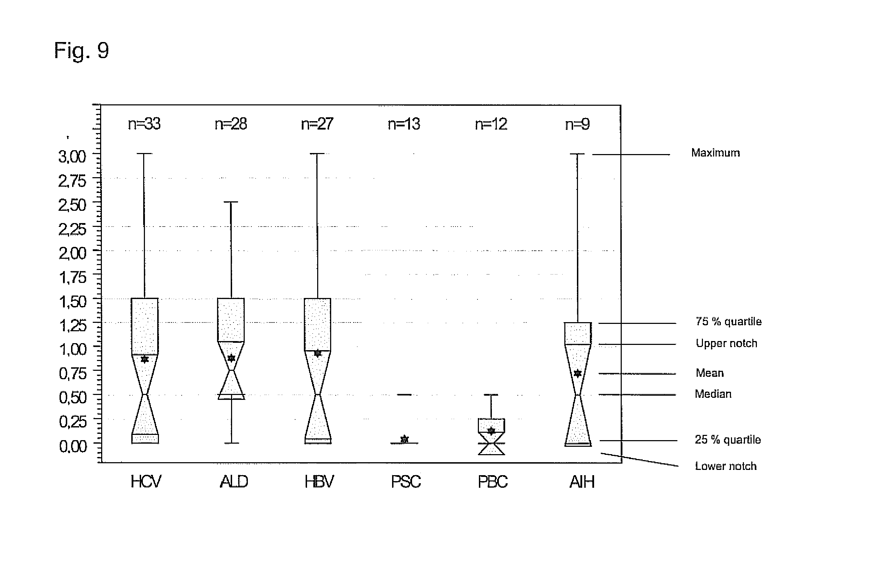

When mean and median values of the semi-quantitative immunohistological score (see methods) were analysed, EpCAM expression was highest in tissues with HBV-infection (mean score: 0.93; median score: 0.5; maximal score: 3; frequency of positive EpCAM staining (+/++/+++): 55.6 %;), ALD (mean score: 0.88; median score: 0.75; maximal score: 2.5; frequency of positive EpCAM staining (+/++/+++): 78.6 %;), and HCV-infection (mean score: 0.86; median score: 0.5; maximal score: 3; frequency of positive EpCAM staining (+/++/+++): 63.6 %). Patients with AIH had an intermediate EpCAM staining (mean score: 0.72; median score: 0.5; maximal score: 3; frequency of positive EpCAM staining (+/++/+++): 55.6 %;). Hepatocellular EpCAM expression was almost absent in patients with the chronic biliary diseases PBC (mean score: 0.13; median score: 0; maximal score: 0.5; frequency of positive EpCAM staining (+/++/+++): 25.0 %;) and PSC (mean score: 0.04; median score: 0; maximal score: 0.5; frequency of positive EpCAM staining (+/++/+++): 7.7%;) (FIG. 9; description of statistical variables is shown in the figure at the right box plot).

**Conclusion.** Tissue samples from patients with chronic liver disease like chronic hepatitis C virus (HCV) and hepatitis B virus (HBV) infection, chronic autoimmune hepatitis (AIH), chronic alcoholic liver disease (ALD) and hepatocellular carcinoma (HCC) were analyzed semi-quantitatively for EpCAM expression in correlation with the stage of liver fibrosis as well as histological and biochemical parameters of necroinflammatory activity. Hepatocytes, which are EpCAM-negative in normal adult liver, showed *de novo* EpCAM expression in many liver tissues from patients with chronic liver diseases. Hepatocellular EpCAM expression was highest in patients with necroinflammatory diseases (HCV and HBV hepatitis, AIH, ALD). Hepatocellular EpCAM expression correlated significantly with histological and biochemical parameters of inflammatory activity and the extent of fibrosis, which was particularly striking in patients with HBV infection. Furthermore, 14.3 % of the HCCs showed EpCAM expression on tumor cells.

The results demonstrate that *de novo* expression of EpCAM occurs only in a fraction of hepatocellular carcinomas (HCCs), but frequently on hepatocytes in chronic necroinflammatory liver diseases. This expression positively correlates with disease activity and fibrosis. Specifically, a correlation of hepatocellular EpCAM neo-expression in chronic necroinflammatory liver disease and fibrosis and necroinflammatory activity has been demonstrated. These findings have implications for upcoming treatment options, such as monoclonal antibodies targeting EpCAM in malignant tumors, and may also apply to some HCCs. As a specific consequence, a fraction of HCCs may represent a valid target for EpCAM directed antibody therapy.

### Example 5: Corroboration of pharmacokinetic predictions using patient data obtained in Phase II study of "Anti-EpCAM"

It was desired to confirm the accuracy of the predictions based on pharmacokinetic modelling (themselves based on pharmacokinetic data obtained from the Phase I study of Anti-EpCAM, see Examples 1 and 2 hereinabove) using actual patient data obtained from a subsequent Phase II study in which Anti-EpCAM was administered according to the invention. This phase II study was an international, open-label, multicenter, randomized, phase II study with two parallel treatment groups. Patients participating in the Anti-EpCAM phase II study were randomized into two groups, the first of which receiving a low dose of Anti-EpCAM (2 mg / kg body weight) administered as explained below, and the second of which receiving a high dose of Anti-EpCAM (6 mg / kg body weight) administered as explained below. In the course of the Anti-EpCAM phase II study, each patient initially received three loading doses of Anti-EpCAM (each either 2 mg/kg body weight or 6 mg/kg body weight, depending on the patient group) spaced one week apart during a loading phase, followed by up to 23 subsequent maintenance doses of Anti-EpCAM (again, either 2 mg/kg body weight or 6 mg/kg body weight, depending on the patient group), wherein single maintenance doses were administered every second week.

According to the pharmacokinetic predictions based on Anti-EpCAM phase I study data, the serum trough level of the first patient group receiving the low dose of Anti-EpCAM would be expected to be on the order of 10 µg/ml (correlating the trough level shown in Fig. 7), whereas the serum trough level of the second patient group receiving the high dose of Anti-EpCAM would be expected to be on the order of 30 µg/ml (correlating with the trough level shown in Fig. 6).

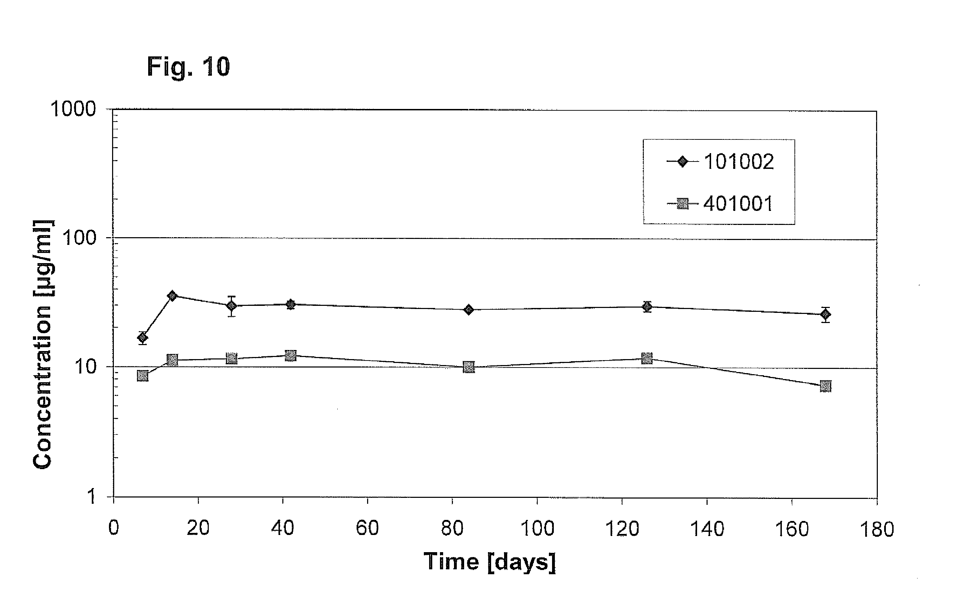

The assay was carried out as follows. 96 well plates were coated with HD4A4 (anti-idiotype Antibody against Anti-EpCAM; 5 µg/ml in a volume of 100 ml) followed by a blocking and washing step. Calibration standards, quality control samples and samples of Anti-EpCAM were added (100 ml in an appropriate dilution) followed by a washing step. Anti-EpCAM bound by HD4A4 was detected with a biotinylated anti-human IgG, again followed by a washing step. Streptavidin was added (0.5 mg/ml in a volume of 100 µl), the 96 well plate was washed again and in a final step 180 µl of pNPP were added. The assay was stopped with 50 µl of 3 M NaOH and measured in an ELISA Reader at 405 and 490 nm. Dilution of low dose samples was performed in a relationship of 1:100. Dilution of high dose samples was performed in a relationship of 1:300. The results are shown in Fig. 10.

Fig. 10 depicts as points the individual trough levels measured for one patient from the low-dosage group (patient number 401001; data points indicated by squares) and another patient from the high-dosage group (patient number 101002; data points indicated by diamonds). The average vertical level of the horizontal line connecting the data points from a respective patient represents the serum trough level observed for that patient. Accordingly, it can be seen that the horizontal line for the high-dose patient 101002 (diamond points) indicates a trough level concentration of Anti-EpCAM in good agreement with the predicted value of 30 µg/ml for this dose (compare horizontal line connecting predicted troughs of graph in Fig. 6). Likewise, the horizontal line for the low-dose patient 401001 (square points) indicates a trough level concentration of Anti-EpCAM in good agreement with the predicted value of 10 µg/ml for this dose (compare horizontal line connecting predicted troughs of graph in Fig. 7).

These data corroborate the accuracy of the trough level predictions by pharmacokinetic modelling based on data obtained during the Anti-EpCAM phase I study with actual patient data obtained during the Anti-EpCAM phase II study. As such, it can be concluded that the assumptions and results of the pharmacokinetic modelling were correct, and that the treatment regimen according to the invention has the effects and advantages elaborated hereinabove.

## Claims

1. A method of treating tumorous disease in a human patient by administering to said patient a human immunoglobulin specifically binding to the human EpCAM antigen, said immunoglobulin exhibiting a serum half-life of at least 15 days, said method comprising the step of administering said immunoglobulin no more frequently than once every week.

2. The method of claim 1, further comprising:
(a) determining, after a period of at least one week following a respective last administration of said immunoglobulin but prior to a respective next administration of said immunoglobulin, the serum level of said immunoglobulin still present in the blood of said patient, thereby obtaining an intermediate serum level value for said immunoglobulin;
(b) comparing said intermediate serum level value for said immunoglobulin with a predetermined serum trough level value for said immunoglobulin;
(c) optionally repeating steps (a) and (b);
(d) effecting the respective next administration if the intermediate serum level value for said immunoglobulin is no more than 15%, preferably 10%, most preferably 5% above the serum trough level value.

3. The method of claim 1 or 2, wherein the magnitude of the dose of said human immunoglobulin administered is set such that, at the end of the intervening time between two respective administrations, the amount of said human immunoglobulin persisting in the serum never drops below the predetermined serum trough level.

4. The method of any of claims 1 to 3, wherein said administering takes place once every two weeks or wherein said administering takes place less frequently than once every two weeks, in particular wherein said administering takes place once every two weeks and wherein the administered dose of said human immunoglobulin remains unchanged from one administration to the next.

5. The method of claim 4, wherein said administering takes place less frequently than once every two weeks and wherein both the administered dose of said human immunoglobulin and the frequency of administration remain unchanged from one administration to the next.

6. The method of claim 4 or 5, wherein the magnitude of the initial and all subsequent doses is determined by pharmacokinetic simulation.

7. The method of any of the above claims, wherein said administering is intravenous, intraperitoneal, subcutaneous, intramuscular, topical or intradermal.

8. The method of any of the above claims, wherein said tumorous disease is breast cancer, epithelial cancer, hepatocellular carcinoma, cholangiocellular cancer, stomach cancer, colon cancer, prostate cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, pancreas cancer, gall bladder cancer, a cancer of the bile duct, esophagus cancer, a cancer of the salivatory glands or a cancer of the thyroid gland, in particular wherein said tumorous disease is prostrate cancer or breast cancer and said human immunoglobulin is administered in a dosage of 1 to 7 mg per kg body weight once every two weeks.

9. The method of claim 8, wherein said human immunoglobulin is administered in a dosage of 2 to 6 mg per kg body weight once every two weeks.

10. The method of any of the above claims, wherein said human immunoglobulin comprises an immunoglobulin heavy chain with an amino acid sequence as set out in SEQ ID NO: 1 and an immunoglobulin light chain with an amino acid sequence as set out in SEQ ID NO: 2.

11. A human immunoglobulin specifically binding to the human EpCAM antigen, **characterized in that** said human immunoglobulin exhibits a serum half-life of at least 15 days after administration to a human patient.

12. The human immunoglobulin of claim 11, wherein the serum half-life is 20 days, 19 days, 18 days, 17 days, 16 days or 15 days, in particular wherein the half-life is 15 days and said human immunoglobulin comprises an immunoglobulin heavy chain with an amino acid sequence as set out in SEQ ID NO: 1 and an immunoglobulin light chain with an amino acid sequence as set out in SEQ ID NO: 2.

13. A pharmaceutical composition comprising the human immunoglobulin of any of claim 11 or 12.

14. Use of a human immunoglobulin specifically binding to the human EpCAM antigen, said human immunoglobulin exhibiting a serum half-life of at least 15 days for the preparation of a medicament for treating a tumorous disease, said medicament being formulated for administration no more frequently than once every week.

15. The use of claim 14, wherein said medicament is formulated for administration no more frequently than once every two weeks, in particular wherein said medicament is formulated for administration every two weeks and the administered dose of said human immunoglobulin remains unchanged from one administration to the next, wherein said medicament is preferably formulated for administration less frequently than once every two weeks, the administered dose of said human immunoglobulin administered being set such that, at the end of the intervening time between two respective administrations, the amount of said human immunoglobulin persisting in the serum never drops below a serum trough level determined to be necessary for therapeutic efficacy.

16. The use of claim 14 or 15, wherein the medicament is formulated for intravenous, intraperitoneal, subcutaneous, intramuscular, topical or intradermal administration and/or wherein the tumorous disease is breast cancer, epithelial cancer, hepatocellular carcinoma, cholangiocellular cancer, stomach cancer, colon cancer, prostate cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, pancreas cancer, gall bladder cancer, a cancer of the bile duct, esophagus cancer, a cancer of the salivatory glands or a cancer of the thyroid gland.
